# EUROPEAN PATENT APPLICATION

(11) **EP 1 568 264 A1**
(43) Date of publication of application: **31.08.2005**
(21) Application number: 04025953.3
(22) Date of filing: 02.11.2004
(51) Int. Cl.: A01C 1/00, A01C 1/02

(54) **Method for improving germination of hard seed by laser beam irradiation and germination improved seed**

(30) Priority: 25.02.2004 JP 2004050547; 22.07.2004 JP 2004214942
(71) Applicant: Takii & Company, Limited, Shimogyo-ku, Kyoto (JP)
(72) Inventor: Watanabe, Yoshihisa, Kusatsu-shi Shiga (JP); Wakisaka, Yasuharu, Jyouyou-shi Kyoto (JP); Hitomi, Haruo, Kameoka-shi Kyoto (JP)
(74) Representative: Strehl Schübel-Hopf & Partner

(57) **Abstract**

Providing a method for improving the germination of plant seeds for use in agriculture, gardening and tree planting on mountains and in forests, where the germination of the seeds is suppressed physically or physiologically due to the hard seed coat (testa or pericarp or the like) as the structures surrounding seed embryos, by secure and uniform treatment of each seed without any damage of seed embryo to produce seeds with improved germination in a stable manner without any loss and without creating problems in the working environment, and providing germination improved seeds. Germination is improved by allowing laser beam to irradiate a part of the seed coat of a plant seed to perforate a part of the seed coat to thereby overcome physical or physiological factors causing the suppression of germination, such as gas permeability and water permeability to seed embryo.

## Description

### Technical Field

The present invention relates to a method for improving the germination of plant seeds with hard seed coats, for use in agriculture and gardening, tree planting on mountains and in forests, and so on.

### Background Art

Some seeds used in agriculture, gardening, and tree planting on mountains and in forests do not swell because water does not permeate through the seed coats (testa and pericarp), so that the seeds can not germinate. This phenomenon is called "property of hard seed", which can cause delayed, low, or inconsistent germination. It can even result in no germination if environmental conditions after seeding are suitable. Poor germination causes crop reduction, inconsistent or delayed crops, and quality deterioration, which are serious economical concerns for producers. Additionally, delayed germination causes very serious problems such as an increased risk of the infection of pathological soil bacteria into seeds or radicles.

As described above, the causes of poor germination due to the structures surrounding seed embryos, such as testa and pericarp (referred to as seed coats hereinafter) are broadly classified into the following two main factors. Generally, these work in a complex manner to cause poor germination.
1) Because of the impermeability of gases such as oxygen and carbon dioxide and water through the seed coats, the transfer of oxygen and water required for germination is blocked so that germination is physiologically suppressed.
2) Because of the exceeding hardness of the seed coats, the appearance of the inner seed embryo is physically blocked, so that germination is suppressed.

In some cases, seed coats containing germination-suppressing agents such as dormant substances or the seeds containing dormant seed embryos as well, may suppress germination. In order to overcome poor germination due to such factors, seeds have traditionally been treated by various physical or chemical treatments. For example, the treatments include the following methods. a) Immersing the seed in water for an extended period of time. b) Abrading and scarifying the seed including a step of rotating seed in a drum attached with a grinding material such as sandpaper on the inner face, or a step of mixing seed with a grinding particulate material such as sand, and rotating the mixture in a drum. c) Treatment at high temperature with wet or dry heat or a physical method with agitation, high pressure, high frequency electric field or ultrasonic wave. d) Chemically treating with an organic solvent, concentrated sulfuric acid and enzyme. e) Temporarily freezing the pericarp in liquid nitrogen and the like and applying pressure to peel off the frozen pericarp. (Publication of Japanese Patent No. 2579822, Publication of JP-A-64-75045, Publication of JP-A-8-70625, Publication of JP-A-8-70626, Publication of JP-A-5-49309; "Norin Shusigaku Soron (General Seed Science for Agriculture and Forestry)", p. 94-101, Shun-ichiro Nakamura, ed., Yokendo, 1985; "Seeds Ecology, Biogeography and Evolution of Dormancy and Germination", p. 101-103, 124, Carol C. Baskin and Jerry M. Baskin, ACADEMIC PRESS, 1998; "Plant Propagation Principle and Practices", 7^{th} edition, 220-226; Hudson T. Hartman, Dale E. Kester, Fred T. Davis Jr., Robert L. Geneve, Prentice Hall 2002; "Principle of Seed Science and Technology", 4^{th} edition, 140-146, Lawrence O. Copeland, Miller B. McDonald, Kluwer Academic Publishers, 2001.)

Although these methods for improving germination have proven to be effective, the methods individually have the following drawbacks.

Method a) of immersion in water for an extended period of time has an only limited and variable effect, although the method can treat a large volume of seed. This method is insufficient for many seeds with particularly hard seed coats. Additionally, this method requires a long period of time and moistening of seeds. For commercial distribution of the resulting seeds, dehydration and drying operations are further necessary, requiring extensive labor together with heating and lighting expenses. Accordingly, this method may cause serious problems commercially. In case that a part of seeds are infected with diseases, such as seed-borne diseases, all the seeds simultaneously treated can become infected.

The method b) of abrading and scarifying seeds including a step of rotating seeds in a drum attached with a grinding material such as sandpaper on the inner face, or a step of mixing seeds with a grinding particulate material such as sand, and rotating the mixture in a drum causes wear of the sand part or the equipment itself along with variation in the abraded and ground states of individual seed coats depending on the treatment scale. Some seeds receive insufficient grinding while others have damaged seed embryos or endosperms due to excessive grinding, leading to severely deteriorated uniformity of germination. Rather than an increase in germination, abnormal germination or no germination occurs. The time needed to grind all the seeds is sometimes longer, although the time depends on the volume of seed to be treated. Thermal deterioration due to abrasion heat during grinding along with physical deterioration due to impact may also potentially inactivate the seed.

By treatment c) at high temperature with wet or dry heat, inactivation of the seed itself readily occurs, while the physical method of agitation, high pressure, high frequency electric field or ultrasonic wave, results in unstable and very slight improvement of germination .

For the chemical treatment d) with organic solvent, conc. sulfuric acid and enzyme, conditions for the treatment should be appropriately be preset, depending on the seed type or the state. Because the concentration of the sulfuric acid solution changes depending on the change of atmospheric temperature, it is difficult to preset the immersion time and the effect is not constant. Additionally, there are occupational safety and environment concerns as well as liquid waste disposal after the treatment. Because the size of each seed, the thickness of each seed coat, and the presence or absence of cracks in each seed vary, it is very difficult to make each seed's coat uniformly fragile. Consequently, insufficient chemical infiltrated seed and excessive chemical infiltrated seed exist concurrently. Accordingly, nearly impossible to preset conditions so all seeds receive the exact treatment each one needs. Should part of the seeds be infected with diseases such as seed-borne disease, the diseases infect all the seeds treated by this method.

Method e) of temporarily freezing pericarp in liquid nitrogen and the like, and then applying pressure to peel off the frozen pericarp is highly effective for improving germination. However the size of each seed and the thickness of the seed coat may vary or may partially vary in each seed. A seed with inner damage is inevitably generated by the method, when the removal of pericarp is intentionally elevated. Therefore, it is very difficult to preset conditions satisfactorily to cope with all seeds. Additionally, the use of liquid nitrogen is a big investment in equipment and material.

As described above, no technique exists for improving the germination of hard seed with poor germination due to the seed coat in a uniformly stable and secure fashion to satisfactorily cope with each seed in the seed lot.

Herein, various investigations have been conducted regarding laser beam irradiation of seed. However, no practical method concerning the improvement of hard seed coat by laser treatment of seed has been found. An experimental example has been reported about laser beam irradiation on tomato seed and corn seed to examine the influence on seed germination or crop ("Tomato Plant Response to Laser Beam Seed Treatment", 451-454, E. Szwonek, A. Felczynska, ISHS, Acta Horticulturae 287, 1990; "He-Ne Laser Beam Irradiation Effect on Germination Response of Corn Plant Seeds" Danila C., Ristici M., Ristici E., [online], [searched on June 7, 2004], on the Internet <see URL: http://ursus.ar.lublin.pl/users/fizarl9/agro/danila.htm>). However, the seeds as the subject of these experiments were not hard seeds. Additionally, the laser beam irradiation effect was for the purpose of effectively promoting the growth through activation at a cellular level via irradiation of photoenergy. Therefore, it is described that the conditions for He-Ne red laser beam irradiation of seed were 1 to 2 mJ/cm²/sec as photoenergy and irradiation times of 240 seconds, 360 seconds and 720 seconds. The photoenergy, at a level not enough to perforate the seed coats, was irradiated for long periods. These experiments are totally different in constitution, purpose and effect from the invention for improving the permeability of seed coat including a step of laser beam irradiation at a high energy density on hard seed coat to instantly perforate the seed coat.

Further, "Space Experiment Module (SEM)-10", NASA, [online], [searched on June 7, 2004], on the Internet <URL: http://sspp.gsfc.nasa.gov/sem/History/history_sem10.html> describes the "Agrilaser" experiment including CO₂ or YAG laser beam irradiation on corn seed and pumpkin seed and the subsequent exposure of the resulting seed to cosmic space to examine the influence on the germination, growth, crop, taste and so forth. In its constitution of laser beam irradiation of each seed, this experiment is similar to the invention. However, the subject seed for this experiment was not hard seed and the perforation by laser beam irradiation is on the premise that the seed should be transferred to cosmic space, where the seed would be exposed to cosmic particles. For this experiment, the inside of seed is put in contact with cosmic particles for possible activation or modification at a cellular level to show the influence on plant germination, growth, food taste and the like. This experiment clearly differs in terms of constitution, purpose and effect from the invention for improving the germination of hard seed with physically or physiologically suppressed germination as the subject, including a step of eliminating or reducing the hard seed property via perforation by laser beam irradiation.

The publication of JP-A-11-56014 also describes a technique for laser beam irradiation on seed. The technique is for the purpose of sterilizing or eradicating pathological bacteria or harmful pests attached to the seed. According to this technique, laser beam irradiates the seed while damage of the seed coat is avoided. Thus, the technique is totally different from the invention in terms of constitution as well as purpose and effect.

### Summary of the Invention

The inventors focused attention on the problem common to the aforementioned techniques for improving the germination of hard seed in the related art, such that individual seeds with a variation in shape or size contained in one seed lot are treated all together. Such a process appears efficient. Unlike industrial products however, seeds in one seed lot have considerable variation in size, shape and seed coat in terms of their thickness. Thus, processes of treating seeds in one lot essentially generates seeds excessively treated or seeds insufficiently treated resulting in loss.

It is an object of the invention to provide a method for improving the germination of hard seed with poor germination derived from the hard seed coat, capable of improving the germination uniformly in a stable and secure manner without creating problems in the working environment, and also to provide seeds with improved germination through rapid and uniform germination and at high germination rates after seeding.

In such circumstances of the related art, the inventors examined a method for appropriately treating each seed one by one as a secure method, though the method would appear to be inefficient. In those cases, variations of properties such as size and shape are observed in each seed lot as described above, so mechanical treatment involves a great deal of difficulty in control and mass production. The inventors examined the problem in detail. Consequently, they found that the water impermeability or gas impermeability of seed could be solved without any damage of inner seed embryo, by allowing a laser beam with a very high energy density capable of melting, evaporating and removing only the uppermost surface of the material, to irradiate each seed one by one to perforate the seed coat. Based on the finding, the inventors created a production technique for producing seed sufficiently satisfactory in terms of germination speed, uniform germination, and germination rate in a stable and uniform manner at a high efficiency without creating problems in the working environment.

The method for improving the germination of hard seed in accordance with the invention includes allowing the laser beam to irradiate a part of the seed coat, (testa or pericarp) as the structure enclosing the seed embryo, perforating the seed coat at one or more positions.

Additionally, it was suggested that producing a larger area of perforated seed coat with a laser beam would be better for reducing the physical suppression of the germination due to the hard seed coat. When a single perforated area, perforation depth or the total perforated area in cases of plural perforation was too large, the storability of the seed after perforation or good growth of seedling from the seed was sometimes affected adversely. Therefore, the inventors found that the problem could be solved by carrying out seed perforation under controls of the single perforated area, or the total perforated area in case of plural perforation, without any adverse effects on the storability of the resulting perforated seed or the growth of seedling from the seed.

According to the method for improving the germination of hard seed in accordance with the invention, specifically, perforation is preferably conducted so that the perforated area at one position is within a range of 0.2 % to 18 % of the total surface area of seed coat. Additionally, the total perforated area of perforated positions is preferably within a range of 0.2 % to 20 % of the total surface area of seed coat.

In accordance with the invention, the laser beam irradiates the hard seed coat as a factor of suppressing the germination to perforate a part of the seed coat so that physical and physiological factors suppressing the germination, such as gas and water permeability, can be overcome or reduced to improve germination. Because the process is done with a laser beam, the process can treat the seed coat appropriately in a short time, with no damage to the inner seed embryo of each seed. Thus, seed with improved germination, and therefore with an ability of high germination after seeding, can be produced in a stable manner with no variation or loss and without creating problems in the working environment.

### Best Mode for Carrying out the Invention

Items relating to the practice of the invention are described in detail below.

Improving the germination of hard seed in accordance with the invention is achieved by using a laser beam at an appropriate intensity (with no damage of inner seed embryo) so it irradiates the seed coat of each seed to securely perforate a part of the seed coat tissue to improve the water and gas permeability of the seed coat.

Numerous research works have traditionally been done about the water impermeability of the hard seed coat. It has been shown that a water-impermeable layer exists in the palisade tissue of seed coat. The palisade tissue is divided into a top part, bright line part and lower layer part. Depending on the researcher and the type of seed, the water-impermeable layer may be classified in a different part among the parts described above. Although it is known that the water-impermeable layer of the hard seed coat of seed exists in the palisade tissue, a different seed type contains the water-impermeable layer in a different part of the palisade tissue. Therefore, the perforation depth into the seed coat with a laser beam in accordance with the invention varies, depending on the subject seed. Actual perforation depth can be determined with preliminary tests. The term "perforation" of seed coat with laser beam irradiation in accordance with the invention means not only the arrangement of the opening (namely, through-hole) to expose the inside of the seed, by perforation through the palisade tissue existing in the seed coat, but also the arrangement of the non-through-hole on the surface of the seed coat, such as groove and recess unpierced through the palisade tissue. Seed with the water-impermeable layer not existing in the deep layer of the palisade tissue but in the superficial layer is preferably perforated just to the depth of the water-impermeable layer, with as little perforation through the whole seed coat as possible. This avoids further damage to the seed embryo inside the seed. The perforated shape is not limited to general holes such as a circular hole but also includes slit-like cracks that are satisfactory as long as they give the same results as described above.

By making such perforations into the seed coat, physiological factors suppressing germination such as gas and water permeability to the seed embryo can be improved. Physical factors suppressing germination of the seed embryo due to the tightness and hardness of the seed coat can be eliminated or reduced.

In almost all of the seeds to which the invention is applicable, the seed coat adheres to inner seed embryo or a very slight space exists between them. Accordingly, each seed should be treated instantly until completion, with a laser beam of an appropriate intensity under appropriate irradiation conditions, so only the seed coat layer is treated without any damage to the inner seed embryo and also to elevate the efficiency. As the laser usable under such conditions, laser oscillator producing a large output in a stable manner is preferable. The laser beam type for use in accordance with the invention includes, but is not limited to, any laser beam type that is adjustable to achieve irradiation on the seed coat without any damage to the inner seed embryo, as described above. Any of the continuous oscillation or pulse oscillation type laser beams may be used with no specific limitation. For the purpose of producing an apparatus for carrying out the invention at low cost, a laser oscillator for use in a commercially available laser beam machine can be used.

The wavelength of the laser beam is determined in a dependent manner on the laser type. Preferably, a wavelength of 0.1 to 15 µm is used. A wavelength of 0.5 to 15 µm is preferred. For example, solid state laser such as ruby (0.69 µm), glass (1.06 µm), and Nd: YAG (1.06 µm), or gas laser such as CO₂ (10.6 µm), Ar (0.51 µm) and excimer (0.15 to 0.35 µm) can be used. Especially preferable is a CO₂ (10.6 µm) laser. A CO₂ laser can oscillate continuously, or in pulse mode efficiently at a high oscillation. Thus, a CO₂ laser is widely applicable and is readily available. In accordance with the invention, therefore, a CO₂ laser is the most readily usable. Additionally, a Nd:YAG laser is also widely used because its optical fiber can be used. The laser is also usable in accordance with the invention.

The position of seed for laser beam irradiation, or the perforation depth in seed coat or the perforation area thereof varies, depending on the seed. These can be determined at a preliminarily test, using a small volume of seed. For a seed with a seed coat with water and gas impermeability as the main cause of the suppression of the germination, for example, the laser beam irradiates a very slight part, or several positions, of the seed coat, so that an effect of improving the germination can generally be achieved via resolution of the water impermeability. For seeds with a hard and tight seed coat as a factor for the physical suppression of germination, some of the seeds can get the effect when the laser beam irradiates the hilum part or the micropyle part, where the seed embryo will emerge; and other such seeds can get the effect at a higher level when the laser beam irradiates plural positions in a relatively wide zone of seed coat making the entire seed coat fragile. Because an adverse effect on the storability of seed and growth of the seedling may arise in cases of perforation in a relatively wide zone, it is preferred that a single perforated area should be within a range of 0.2 % to 18 % of the whole surface area of seed coat, while the total perforated area should be within a range of 0.2 to 20 % of the surface area of seed coat. The single perforated area within a range of 0.05 mm² to 5.0 mm² frequently leads to a preferable result, although the result depends on the seed type or size.

Various methods for the irradiation of laser beam use on seed are applicable. For example, seed can be irradiated in a many-sided way by reflecting the laser beam. Satisfactorily, irradiation of laser beam use on seed may be done by transferring the seed while the laser beam source is fixed, or by transferring the source while seed is fixed. Both of the two may be fixed or transferred, or one of them may be fixed while the other may be transferred.

The irradiation intensity of the laser beam on seed has a relation to perforation depth and differs depending on the seed as an irradiation subject. Any intensity producing an action on the seed coat without any damage to the inner seed embryo may be satisfactory with no specific limitation. The intensity varies depending on the subject seed and can be determined at a preliminary test on a small volume. Specific adjustments are done as follows. The frequency of laser oscillation, the output of laser beam, and the irradiation time can be adjusted, while the focus of laser beam is adjusted with a converging lens to adjust the converging dose.

The hard seed to which the invention is applicable, is any seed with poor germination such as non-uniform germination, delayed germination or no germination, due to the main cause of the hardness of seed coat (testa or pericarp) as the structure surrounding seed embryo.

For example, the seed includes those of the Chenopodiaceae family, such as spinach (Spinacia oleracea), swiss chard (Beta valgaris), sugar beet (Beta valgaris) and table beet (Beta valgaris) ; those of the Convolvulaceae family, such as morning glory (Ipomoea nil), moonflower (Calonyction aculeatum), and engtsai (Ipomoea aquatica); those of the Cannaceae family, such as Canna (Canna generalis); those of the Geraniaceae family, such as geranium (Geranium sp.) and pelargonium (Perargonium sp.); those of the Fabaceae family , such as pea (Pisum sativum), fava bean (Vicia faba), haricot (Phaseolus sp.), soybean (Glycine max), kudzu (Pueraria lobata ohwi), crotalaria (Crotalaria sp.), lupine (Lupinus sp.) or lupinus (Lupinus sp.), cowpea (Vigna sinensis), sword bean (Canavalia gradiata), subclover (Trifolium sp.) and alfalfa (Medicago sativa); those of the Myrtaceae family, such as eucalyptus(Eucalyptus sp.); those of the Malvaceae family, such as okra (Abelmoschus esculentus), sunset hibiscus (Abelmoschus manihot), hibiscus (Hibiscus sp.) and cotton (Gossypium sp.); those of the Zingiberaceae family, such as shell ginger (Alpinia sp.); those of the Primulaceae family such as cyclamen (Cyclamen sp.) and primula (Primula sp.); those of the Verbenaceae family, such as verbena (Verbena x hybrida); those of the Rose family , such as rose (Rose sp.) and strawberry (Fragaria sp.); those of the Cucurbitaceae family, such as watermelon (Citrullus lanatus), sponge gourd (Luffa cylindrica) and lagenaria (Lagenaria siceraria) ; those of the Asteracea family , such as burdock (Arctium lappa), crown daisy (Chrysanthemum coronarium), and sunflower (Helianthus annuus); those of the Liliaceae family, such as asparagus (Asparagus officinalis, Asparagus plumosus); those of the Nymphaeaceae family, such as lotus (Nelumbo nucifera). Tree and shrub seeds with hard-seededness are also included in applicable seeds to the invention. For example, those of the Anacardiaceae family , such as Japanese sumac (Rhus verniciflua Stokes) and wax tree (Rhus succedanea L.); those of the Pinaceae family, such as Japanese white pine (Pinus parviflora), Japanese black pine (Pinus thunbergii), and Japanese red pine (Pinus densiflora); and those of the Juglandaceae family, such as walnut (Juglans sp.).

The seeds of these plants germinate poorly often due to the seed coats (testa and pericarp). The germination can be improved sufficiently by the method of the invention.

The seed after the completion of irradiation by the laser beam may be treated with a priming process or forcing of the germination process. A hard seed with problems in terms of the water and/or gas permeability of the seed coat is treated by irradiation of a laser beamprior to a process of water supply to the seed, such as priming and forcing of the germination process, so that the effect of the priming process and the forcing of the germination process can be improved. Herein, the priming process means a process of treating seed with water, time and temperature satisfactory for the initiation of the metabolic action in the seed toward germination but insufficient for germination. The priming process can be done by the method described in Japanese Patent No. 3151471 or 3205896. Additionally, the forcing of germination process means a treatment for sprouting before seeding, by supplying water to the seed. The forcing of the germination process can be done by the method described in JP-A-10-117511.

After the irradiation by laser beam is done in accordance with the invention, or after processes such as a priming process are additionally done, the seed can be treated by coating granulation, film coating, seed tape process and seeding sheet process. Herein, coating granulation means molding and granulation of seed by enclosing the seed with a granulation agent in a pill form, using a granulation agent (excipient) such as clay and talc and a moistening agent such as water, for the purpose of mechanical seeding. For example, the method described in Japanese Patent No. 2520309, JP-A-10-225207 or JP-A-10-225208 can be used. Further, the film coating process means coating the surface of seed with a thin film, using a coating material prepared by dissolving and dispersing an agricultural chemical or the like in an aqueous solution of a water-soluble or hydrophilic polymer. For example, the method described in Japanese Patent No. 1719604 or JP-A-11-146707 can be used therefor. The seed tape process means sealing seed beforehand at a given interval with a tape made of a water-soluble material or a biodegradable material for the purpose of readily seeding of seed at a given interval. For example, the method described in JP-UM-A-05-007013 can be used therefor. Additionally, the seeding sheet process means attaching seed at a given interval on a sheet made of a water-soluble material or a biodegradable material and suitably coped with the seeding area for the purpose of readily seeding on a cultivation tray or the like. For example, the method described in JP-A-11-332312 can be used.

If necessary, the seed may be treated with a process of coating with a fungicide and the like for preventing and eradicating damping-off disease, subsequent to the method for improving the germination in accordance with the invention. For example, the process of preventing epidemics can be done by the method described in "Ecology and control of Seed Born Diseases (Shusi Densensei Byo no Seitai to Boujyo)" (edited by Kan-ichi Ohata et al., issued by Japan Plant Protection Association, 1999, p. 66-68).

By the method and treatment for improving the germination in accordance with the invention, further, laser beam irradiates the whole seed coat, after or before the irradiation of laser beam on a part of seed coat for perforation, to modify or eliminate substances suppressing germination as contained in the whole seed coat, to remove the factors causing poor germination derived from the substances suppressing germination. Substances suppressing the germination as contained in seed coat can be modified or eliminated by the irradiation by the laser beam, without apparent detectable change to the surface of seed coat. Thus, poor germination due to the substances suppressing germination can be improved. For irradiation by the laser beam on the whole seed coat, the laser beam is satisfactorily dispersed by shifting the focus of the laser beam to enlarge the irradiated zone. The laser beam intensity required for modifying and eliminating such substances suppressing germination is sufficiently low compared with the intensity in case of the perforation described above. Adverse influences on the storability of seeds and growth of seedling after irradiation can be suppressed, although the laser beam then irradiates the whole seed coat.

By the method and treatment for improving the germination as described above, a laser beam at an appropriate intensity so as not to cause any damage to the inner seed embryo, irradiates each seed coat to securely perforate a part of the seed coat as a factor causing the suppression of germination. Therefore, an effect on uniform germination in a stable manner can be obtained without any adverse effects on germination.

Each seed is instantly treated by the irradiation of the laser beam in accordance with the invention, so the time required for treating each seed lot in its entirety can be shortened.

The treatment with the irradiation by a laser beam can be done without any contact to seed, compared to a seed that is holed with a drill or the like. Therefore, the infection of other seeds with a disease can be suppressed, even when a seed afflicted with the disease is contained therein. Because each seed is treated one by one as described above, and the time required for the treatment is short, the infection of other seeds with the disease can be reduced.

The irradiation treatment with a laser beam can give the seed coat a sufficient effect to improve the germination unless the seed is of an extremely large size variation. A laser beam can have an energy corresponding to a distance from the focus, even in front of or behind the focus, when the height of seed coat to be treated shifts from the focus of laser beam due to the size variation. When seeds are automatically treated mechanically using a drill as described above, the tip of the drill can never be put in contact to the seed coat when the height of the seed coat to be treated varies due to the size variation, so that such seed coat can never be treated. From such standpoint, the irradiation treatment of the invention is absolutely different from the mechanical process.

Even when a seed coat has an undesirable crack, for example, the possibility of the irradiation of the crack with a laser beam is low during the irradiation process of a part of the seed coat with laser beam. Excess infiltration of chemical substances into a part of seeds to deteriorate the germination never occurs, which is observed in the case of chemical processes with the chemical substances in the related art. Even if laser beam irradiates such a crack, the crack part recesses at a corresponding depth from the original surface of the seed coat, so the crack is irradiated at a part with a shift from the focus of the laser beam and any excess laser treatment can be reduced.

Because the treatment is done with the irradiation by a laser beam, there are no work environment problems as observed in the case of processes using chemical substances.

Laser beam irradiates a part of the seed coat for perforation, in particular, only the part treated is the part needed to make a contribution to the improvement of germination. Because the remaining part is left as is without the treatment, any damage to the seed embryo due to the irradiation by the laser beam can be suppressed as much as possible. In the case of collectively treating each seed lot in the related-art manner (for example, the grinding process b)), the whole seed coat is treated so that such damage of the seed embryo reaches its entirety. Thus, the method of the invention apparently differs from the related-art processes.

### Examples

Specific examples are described below. However, the invention is never limited by these examples.

Laser beam irradiation on the seed in the examples was done using a commercially available CO₂ laser marker ML-G9300 (manufactured by KEYENCE) for printing. For actual laser beam irradiation on seed, ML-G9300 was singly used for tests in Test Examples 1 and 3. A laboratory ack was used as a working table. Seeds of tens to several hundreds were placed on a paper piece with a block frame line drawn thereon within the zone of the marking area (110 mm²) of ML-G9300, to manually irradiate with the laser beam. For tests in Test Examples 2, 4 and 5, a series of courses of seed supply, positioning, laser beam irradiation and recovery was automatically carried out, using an original apparatus prepared by integrating a laser controller unit into units for example for continuous seed supply, automatic alignment, automatic transfer and automatic discharge, together with ML-G9300.

In accordance with the invention, further, conditions such as laser beam irradiation intensity responsible for the perforation depth into seed coat varied depending on the seed. Therefore, such conditions were determined appropriately at preliminary tests. Individual irradiation conditions were adjusted by regulating the mean output (30 W) of the laser oscillator (laser power = 0 to 100 % range) and the shift speed (scan speed of 1 to 12,000 mm/s) of the laser focusing spot. The irradiation conditions varied depending on the preset frequency of the Q switch of the laser oscillator. In the present examples, the frequency was fixed at 25kHz (pulse oscillation) in view of the specifications of the units used.

### [Test Example 1: Examples 1 and 2 and Comparative Examples 1 and 2]

Canna (Canna sp.) seed with ready occurrence of poor germination due to the hard seed coat with water impermeability due to the hard-seededness of the coat was treated for the improvement of germination via the irradiation by the laser beam. The seed was of the variety "Tropical Rose" from Takii & Co., Ltd.. The irradiation with the laser beam on the seed coat was done as follows. The laser beam irradiated the seed coat of each seed one by one, while the focus of the laser beam was set on the surface of the seed coat. The scanning speed was set at 300 mm/s and the laser power was set at 45 %. Seeds with improved germination were obtained where a circular perforated hole of a diameter of about 0.8 mm was prepared at one position (Example 1) and three positions (Example 2).

As Comparative Example 1, seeds of the same lot before the treatment were treated for improving the germination with sulfuric acid. In that case, the seeds were immersed in 36N sulfuric acid for 20 minutes according to the routine method, rinsed in tap water for 5 minutes and subsequently dried in air at 40 °C for 2 hours (Comparative Example 1).

The obtained seeds with improved germination (Examples 1 and 2 and Comparative Example 1) and the seed of the same lot before the treatment (Comparative Example 2) were tested for their germination. The results are shown in Table 1. The germination test was carried out by seeding the seeds in a cultivation soil, maintaining the seeds at a temperature around 20 to 25 °C and examining the days from the seeding to germination over time.

**Table 1**

| Results of germination test on soil of Canna seed (Tropical Rose) after irradiation with a laser beam (100 seeds per each Example were tested) | | | | | | |
|---|---|---|---|---|---|---|
| | Treatment | Germination rate (%) | | | | Note |
| | | 9 days later | 10 days later | 14 days later | 21 days later | |
| Example 1 | Laser treatment | 4 | 42 | 88 | 98 | - |
| Example 2 | Laser treatment | 16 | 48 | 88 | 94 | - |
| Comparative Example 1 | Acid treatment | 0 | 10 | 40 | 75 | Abnormal seedling at 6 % |
| Comparative Example 2 | No treatment | 0 | 2 | 2 | 22 | - |

Compared with the non-treated seed of Comparative Example 2, prominent effects on the improvement of all of the germination speed, uniformity and germination rate were observed in the seeds of Examples 1 and 2 in accordance with the invention. An effect on the improvement of germination was observed in the seed of Comparative Example 1 as treated with the acid by the routine method, but was not superior to the improvement in accordance with the invention. Abnormal seedling with young roots or cotyledons partially damaged with the acid was observed in 6 % in Comparative Example 1.

Further, the inner state of water absorption was observed in the individual non-germinated seeds. Some of the insides of the non-germinated seeds of Comparative Example 1 as treated by the routine method and of Comparative Example 2 with no treatment could not absorb water. Accordingly, the individual seeds with the insides still being hard were numerous under observation. In other words, it was found that the acid treatment by the routine method could not completely improve the water impermeability of seed coat and that additional treatment with acids was required. However, germinated seedlings were affected by the treatment to an excess level so that seedlings infiltrated with the acid amounted to 6 %. Accordingly, it was shown that the seeds cannot be additionally treated. This may be due to the existence in the "variation" of the thickness of seed coat and the existence of seeds with seed coats with slight cracks and small holes within the seed lot, so that these individuals might be damaged by the infiltration of the acid. It can be said in a practical sense that all the seeds cannot be uniformly treated equally by the routine method.

In the non-germinated seeds of Example 1, even after the irradiation treatment with laser beam in accordance with the invention, the insides of all the seeds absorbed water and swelled. It could accordingly be verified that the water impermeability of seed coat was completely improved. Seeds with such variation as described above within the seed lot could be treated uniformly.

### [Test Example 2: Examples 3 through 13 and Comparative Example 3]

Canna (Canna sp.) seed with ready occurrence of poor germination because of the hard seed coat with water impermeability due to the hard-seededness was irradiated with the laser beam to perforate the seed at a level enough to almost make through-holes in the seed coat under perforation conditions shown in Table 2, while the preset conditions about the number of perforated positions on each seed or the diameter of perforated hole thereon were variable, to obtain seeds with improved germination. The seed was of the variety "Tropical Yellow" from Takii & Co., Ltd. The irradiation with the laser beam on the seed coat was done at a scanning speed fixed to 500 mm/s while the laser power was fixed to 50 %.

The term "single perforated area" means the value of the area of a single perforated hole, namely a circular perforated hole as determined by calculation. The term "single perforation ratio" means the percentage of the single perforated area to the surface area of seed coat and was calculated by the formula: (single perforated area/surface area of seed coating) × 100. The term "total perforated area" means the value of the sum of the areas of all the perforated holes as calculated by the formula: single perforated area x number of perforated holes. The term "total perforation ratio" means the percentage of the total perforated area to the surface area of seed coat and was calculated by the formula: (total perforated area / surface area of seed coat) × 100. The surface area of seed coat was calculated by measuring the mean particle size of Canna seed and defining its shape as sphere.

The obtained seeds with improved germination (Examples 3 through 13) and the seed before the treatment of the same lot (Comparative Example 3) were tested for germination. The results are shown in Table 3. The germination test was carried out by the same method as in Test Example 1.

**Table 2**

| Conditions for laser beam irradiation on Canna seed (Tropical Yellow) | | | | | | |
|---|---|---|---|---|---|---|
| | Number of perforated holes (positions) | Diameter of perforated hole (mm) | Single perforated area (mm²) | Single perforation ratio (%) | Total perforated area (mm²) | Total perforation ratio (%) |
| Example 3 | 1 | 0.2 | 0.03 | 0.1 | 0.03 | 0.1 |
| Example 4 | 3 | 0.2 | 0.03 | 0.1 | 0.09 | 0.3 |
| Example 5 | 1 | 0.3 | 0.07 | 0.2 | 0.07 | 0.2 |
| Example 6 | 1 | 0.5 | 0.2 | 0.6 | 0.2 | 0.6 |
| Example 7 | 1 | 1 | 0.79 | 2.2 | 0.79 | 2.2 |
| Example 8 | 2 | 1 | 0.79 | 2.2 | 1.57 | 4.3 |
| Example 9 | 4 | 1.5 | 1.77 | 4.9 | 7.1 | 19.6 |
| Example 10 | 2 | 2 | 3.14 | 8.7 | 6.3 | 17.45 |
| Example 11 | 3 | 2 | 3.14 | 8.7 | 9.42 | 26.2 |
| Example 12 | 1 | 2.5 | 4.91 | 13.6 | 4.91 | 13.6 |
| Example 13 | 1 | 3 | 7.07 | 19.1 | 7.07 | 19.1 |
| Comparative Example 3 | 0 | - | - | - | - | - |

**Table 3**

| Results of cultivation test on soil of Canna seed (Tropical Yellow) after laser beam irradiation (200 seeds of each Example were tested) | | | | | | | |
|---|---|---|---|---|---|---|---|
| | Treatment | Number of perforated holes (positions) | Single perforation ratio (%) | Total perforation ratio (%) | Germination rate (%) | | |
| | | | | | 10 days later | 14 | 21 |
| Example 3 | Laser treatment | 1 | 0.1 | 0.1 | 28 | 51 | 58 |
| Example 4 | Laser treatment | 3 | 0.1 | 0.3 | 25 | 46 | 63 |
| Example 5 | Laser treatment | 1 | 0.2 | 0.2 | 40 | 80 | 85 |
| Example 6 | Laser treatment | 1 | 0.6 | 0.6 | 48 | 83 | 85 |
| Example 7 | Laser treatment | 1 | 2.2 | 2.2 | 55 | 85 | 91 |
| Example 8 | Laser treatment | 2 | 2.2 | 4.3 | 51 | 80 | 93 |
| Example 9 | Laser treatment | 4 | 4.9 | 19.6 | 49 | 79 | 84 |
| Example 10 | Laser treatment | 2 | 8.7 | 17.45 | 49 | 82 | 88 |
| Example 11 | Laser treatment | 3 | 8.7 | 26.2 | 47 | 67 | 68 |
| Example 12 | Laser treatment | 1 | 13.6 | 13.6 | 50 | 76 | 86 |
| Example 13 | Laser treatment | 1 | 19.1 | 19.1 | 38 | 47 | 61 |
| Comparative Example 3 | No treatment | 0 | - | - | 0 | 6 | 27 |

Compared with Comparative Example 3 with no treatment, effects of improving all the seeds of Examples 3 through 13 as treated in accordance with the invention, in terms of faster germination, uniformity and germination rate, were observed. Meanwhile, comparison was made in Examples 3 through 13. A relatively low effect was observed in the seeds with smaller single perforated areas in Examples 3 and 4, while it was observed that the seed with a very large single perforated area of Example 13 and the seed with a relatively large total perforated area of Example 11 were likely to partially have slight delay of the growth and/or mild damage on the seedlings after germination. The non-germinated seed of Example 3 was cut to observe the inner state of water absorption. It was observed that some of the individual seeds swelled insufficiently.

Under conditions for the larger single perforated area or larger total perforated area as in Examples 13 and 11, alternatively, it was observed that the vigor of the seeds was reduced at a relatively early stage in the subsequent storage. Therefore, it was suggested that excess removal of seed coat, essentially existing for seed protection might have some influence on seed. Based on these contexts, it is suggested that a more preferable embodiment of the invention is carried out under conditions that a single perforated area per one position is within a range of 0.2 to 18 % of the total surface area of seed coat (about 0.05 to 5 mm² per single perforated area) and that the total area of perforated positions in case of perforations at plural positions is within a range of 0.2 to 20 % of the total surface area of seed coat.

### [Test Example 3: Examples 14 and 15 and Comparative Examples 4 through 7]

Morning glory (Ipomoea nil) seed and Green Engtsai (Ipomoea aquatica) seed, both with ready occurrence of poor germination because of their hard seed coat with water impermeability due to the hard-seededness of the coat, were treated by the process of improving germination with the irradiation with a laser beam in accordance with the invention. The seeds were of the variety "Beni Chidori" for the Morning glory seed from Takii & Co., Ltd. and of the variety "Ensai" for the Green Engtsai seed from the Takii & Co., Ltd.

The irradiation with the laser beam on the Morning glory seed was done at a scanning speed of 500 mm/s and at a laser power of 60 %. Other conditions were the same as in Example 1. The seed coat of each seed was spot-irradiated at two positions, to obtain a seed with improved germination, where two perforated holes of a diameter of about 1 mm were made (Example 14).

The irradiation with the laser beam on the Green Engtsai seed was done at a scanning speed of 800 mm/s and at a laser power of 60 %. Other conditions were the same as in Example 1. The seed coat of each seed was spot-irradiated at one position, to obtain a seed with improved germination, where one perforated hole of a diameter of about 1 mm was made (Example 15) .

Seeds before the treatment of each of the lots of Morning glory and Green Engtsai were treated by abrasion and scarification for improving germination. Using a commercially available carrot hair remover (manufactured by Westrup) with a rasp on the interior wall of the cylinder of a cylindrical shape according to the routine method, each seed coat was abraded and scarified (Comparative Examples 4 and 5) .

The seeds treated for improving germination (Examples 14 and 15 and Comparative Examples 4 and 5) and seeds of the same lots before the treatment (Comparative Examples 6, 7) were tested for germination. The results are shown in Tables 4 and 5. The germination test was done by the same method as in Test Example 1.

**Table 4**

| Results of soil germination test of Morning glory seed after laser beam irradiation (100 seeds of each Example were tested) | | | | |
|---|---|---|---|---|
| | Treatment | Germination rate (%) | | |
| | | 5 days later | 10 | 14 |
| Example 14 | Laser treatment | 75 | 85 | 90 |
| Comparative Example 4 | Abrasion and wear | 70 | 80 | 85 |
| Comparative Example 6 | No treatment | 15 | 48 | 67 |

**Table 5**

| Results of soil germination test of Green Engtsai seed after laser beam irradiation (100 seeds of each Example were tested) | | | | |
|---|---|---|---|---|
| | Treatment | Germination rate (%) | | |
| | | 5 days later | 8 | 14 |
| Example 15 | Laser treatment | 85 | 90 | 90 |
| Comparative Example 5 | Abrasion and wear | 13 | 32 | 65 |
| Comparative Example 7 | No treatment | 15 | 20 | 55 |

### [Test Example 4: Examples 16 and 17 and Comparative Examples 8 and 9]

Triploid watermelon (Citrulls lanatus) seed with ready occurrence of poor germination because of the hard seed coat with water impermeability due to the hard-seededness of the coat was treated by the process of improving germination with the irradiation of laser beam in accordance with the invention. The seed was of the variety "T-173" for the triploid watermelon from Takii & Co., Ltd.

The irradiation of with the laser beam on the seed coat was done at a scanning speed of 1,000 mm/s and at a laser power of 40 %. Laser beam irradiated the seed coat of each seed, to obtain a seed with improved germination, where a hole of a diameter of about 1 mm at a level not enough to make any through-hole through the seed coat layer was made at three positions (Example 16).

Additionally, the seed with improved germination from Example 16 was treated with the priming process by the method described in Japanese Patent No. 3205896 (Example 17). More specifically, the laser-treated seed was mixed with an equal weight of silica hydrogel of a particle size of 75 to 500 µm and a water retention ratio of 350 % by weight. The resulting mixture was treated in a room at 20 °C for 5 days, for priming (hydration treatment).

The obtained seeds with improved germination (Examples 16 and 17), the seed of the same lot before the treatment but after the priming treatment alone (Comparative Example 8) and the seed of the same lot before these treatments (Comparative Example 9) were tested for germination. The results are shown in Table 6. The germination test was done by the same method as in Test Example 1.

**Table 6**

| Results of soil germination test of triploid watermelon seed after laser beam irradiation (200 seeds of each Example were tested) | | | | | |
|---|---|---|---|---|---|
| | Treatment | Germination ratio (%) | | | |
| | | 5 days later | 7 | 10 | 14 |
| Example 16 | Laser treatment | 58 | 83 | 87 | 90 |
| Example 17 | Laser treatment + priming | 75 | 87 | 89 | 89 |
| Comparative Example 8 | No treatment + priming | 59 | 72 | 75 | 80 |
| Comparative Example 9 | No treatment | 0 | 45 | 53 | 57 |

It was confirmed that compared with Comparative Example 9 with no treatment, the germination of the seed of Example 16 was greatly enhanced.

Further, Example 17 was compared with Comparative Examples 8 and 9. Although priming alone is effective for improving germination, the effect of the priming treatment on the improvement of germination can be elevated more by carrying out the treatment of the invention.

### [Test Example 5: Examples 18 and 19 and Comparative Examples 10 and 11]

Luffa (Luffa cylindrica) seed with ready occurrence of poor germination because of the hard seed coat with water impermeability due to the hard-seededness of the coat was treated by the process of improving germination with irradiation with the laser beam in accordance with the invention. The seed was of the variety "Futo Hechima" from Takii & Co., Ltd. The irradiation with the laser beam on the seed coat was done at a scanning speed preset to 1,200 mm/s and at a laser power preset to 30 %. Laser beam irradiated the seed coat of each seed, to obtain a seed with improved germination, where a hole of a diameter of about 0.5 mm was perforated at two positions (Example 18).

The obtained seed with improved germination was treated by film coating by the method described in JP-A-11-146707 (Example 19). More specifically, a coating material of 30 parts by weight of titanium oxide of a mean particle size of 0.25 µm, one part by weight of methyl cellulose (an aqueous 2 % solution thereof was at a viscosity of 25 cp), 69 parts by weight of water and 5 parts by weight of an aqueous pigment was prepared. Using a coating pan of a side aeration type, the coating material was sprayed and coated on the seed after the laser treatment, at 7 % by weight to the seed weight by a general method.

The seed coat at the hilum part of each seed of the same lot before the treatment was carefully cut and removed by hand with a nail clipper to obtain a seed with improved germination (Comparative Example 10). The radicle thereof first appears from the hilum part. For 200 seeds, the removal of the hilum part by hand required more than one hour, while the removal by the laser treatment was completed from one to two minutes.

The obtained seeds with improved germination (Examples 18 and 19), the seed prepared by hand of Comparative Example 10, and the seed of the same lot before the treatment (Comparative Example 11) were tested for germination. The results are shown in Table 7. The germination test was done by the same method as in Test Example 1.

**Table 7**

| Results of soil germination test of Luffa seed after laser beam irradiation (200 seeds of each Example were tested) | | | | | |
|---|---|---|---|---|---|
| | Treatment | Germination ratio (%) | | | |
| | | 2 days later | 3 | 4 | 7 |
| Example 18 | Laser treatment | 0 | 38 | 84 | 94 |
| Example 19 | Laser treatment + film coating | 0 | 40 | 83 | 92 |
| Comparative Example 10 | Manual treatment with nail clipper | 0 | 42 | 86 | 92 |
| Comparative Example 11 | No treatment | 0 | 0 | 0 | 15 |

It was confirmed that compared with Comparative Example 11 with no treatment, the germination of the seed of Example 18 as treated in accordance with the invention was greatly improved. Even compared with Comparative Example 10 where the removal of the hilum part of the seed coat by hand required more than one hour, the same effect on the improvement on germination was observed in Example 18. Absolutely the same effect was confirmed in Example 19 treated with film coating.

As described above, the invention can effectively be utilized for improving the germination of hard seed for use in agriculture, gardening and tree planting on mountains and in forests.

## Claims

1. A method for improving the germination of a hard seed, including a step of allowing laser beam to irradiate a part of a seed coat surrounding a seed embryo in the hard seed for perforation at one or more positions in the seed coat.

2. A method for improving the germination of a hard seed according to claim 1, including a step of perforation at one or more positions, where the perforated area at one position is within a range of 0.2 to 18 % of the total surface area of the seed coat.

3. A method for improving the germination of a hard seed according to claim 1 or 2, including the step of perforation where the total area of perforated positions is within a range of 0.2 to 20 % of the total surface area of the seed coat.

4. A method for improving the germination of a hard seed according to any one of claims 1 through 3, including a step of treating the seed after laser beam irradiation with at least one of priming process, forcing of germination process, coating granulation, film coating, seed tape process and seeding sheet process.

5. A germination improved seed, as obtained by a method according to any one of claims 1 through 4.
